(19) Europäisches Patentamt / European Patent Office / Office européen des brevets

(11) **EP 3 734 548 A1**

(12) **EUROPEAN PATENT APPLICATION**
published in accordance with Art. 153(4) EPC

(43) Date of publication:
**04.11.2020 Bulletin 2020/45**

(51) Int Cl.:
*G06T 7/00* (2017.01)

(21) Application number: **18896144.5**

(86) International application number:
**PCT/JP2018/048095**

(22) Date of filing: **27.12.2018**

(87) International publication number:
**WO 2019/131858 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2017 JP 2017254506**
**16.08.2018 JP 2018153066**

(71) Applicant: **Normee Limited**
**Tokyo 103-0015 (JP)**

(72) Inventors:
• **IWATA Eizaburo**
**Tokyo 103-0015 (JP)**
• **IWAKI Yasuharu**
**Tokyo 103-0015 (JP)**

(74) Representative: **Harrison IP Limited**
**3 Ebor House**
**Millfield Lane**
**Nether Poppleton, York YO26 6QY (GB)**

(54) **PERSONAL AUTHENTICATION METHOD AND PERSONAL AUTHENTICATION DEVICE**

(57)    One of the objects of the present invention is to perform One to many Authentication authenticating an individual at high speed and with high accuracy while using large-scale registration data. The identification unit 4 uses the identification query data to identify a plurality of registered identification registration data, thereby identifies one or more verification registration data to be used in verification. The verification unit 3 performs 1:1 Verification with the identified one or more verification registration data using the degree of similarity with the verification query data. Thereby, a unique individual can be authenticated.

Fig. 1

Template Image Acquisition Device — 2

Authentication Image Acquisition Device — 1

Identification Unit — 4

Verification Unit — 3

EP 3 734 548 A1

## Description

## Technical Field

The present invention relates to a technique for authenticating an individual. More specifically, the present invention provides a technique for performing one to many Authentication which can authenticate a unique individual at high speed and with high accuracy from large-scale registration data.

## Background Art

In the 1:N Identification, a user has only to input his own biometric information, so that a highly convenient authentication system can be realized. However, the 1:N Identification has a problem that the authentication accuracy deteriorates as the number N of registered users increases. In order to solve this problem, a multi-modal authentication technique has been proposed in which a plurality of sets of biometric information (fingerprint, face, voice, etc.) are combined to identify a user with improved authentication accuracy. However, in this case, the user is required to input a plurality of kinds of biometric information, and the convenience as the original advantage of 1:N Identification is reduced. Therefore, it is important to improve the authentication accuracy while keeping the required times of inputting the biometric information minimum. In addition, different sensors are required to acquire a plurality of kinds of biometric information such as fingerprints, faces, and voices, which increases the requirement for the device of biometric authentication system.

For example, JP2010-152706A (Patent Document 1 below) proposes two-factor authentication using a combination of a palm vein pattern and a palm contour image. In this method, if there are two or more matching results based on the vein pattern, erroneous identification is reduced by narrowing down the results based on the difference feature of the palm contour image.

In addition, in International Publication WO2013 / 1365353 (Patent Document 2 below), two-step authentication is proposed in which the first template data corresponding to a palm print pattern and the second template data corresponding to a vein pattern are prepared. Then the first authentication data corresponding to the palm print pattern and the second authentication data corresponding to the vein pattern are acquired, and the authentication is performed in two steps.

Since the techniques described in Patent Documents 1 and 2 use two or more sets of biometric information called multimodal biometrics, the authentication accuracy can be improved. However, since these are methods based on 1:1 Verification, for example, when registration data is huge, it is necessary to perform verification process using a plurality of sets of biometric information on all the registration data. In such technique verifying all registered data, the process time becomes enormous when the number of registered data is huge. That is, these techniques cannot be used in a practical system for huge registration data.

As a method of increasing the number of N in 1:N Identification, in JP2010-191573 (Patent Document 3 below), when finger vein data is registered in a database, it is proposed to group the finger vein data by finger fold data and to register each group in order to reduce the number of verifications. In this method, n databases are created by grouping the finger vein data into n groups by finger fold data. However, in order to acquire the finger vein data and the finger fold data, it is necessary to photograph twice, which impairs the convenience of the user. In addition, the accuracy of grouping by fold data is low, and the number of groups is at most two or several groups. Thus, it is impossible to cope with a huge number of registered data as used in a payment system.

By the way, the present inventor has proposed the technique in International Publication WO2012 / 014300 (Patent Document 4 below). In this technique, two visible light cameras are arranged to face each other. Then, an individual can be authenticated by

(1) generating the first authentication data from the palm print pattern in the first image acquired by the first camera,
(2) generating the second authentication data from the pattern of the vein in the second image acquired by the second camera, and
(3) using the degree of similarity (correlation between images) with the first authentication data and the first template data, and the degree of similarity with the second authentication data and the second template data.

This document describes an example of generating the above-mentioned authentication data and template data using Radon transform. The verification using data generated by the Radon transform has the advantages that it is robust against rotation and scaling and has excellent resistance to parallel movement. However, this technique was premised on 1:1 Verification.

For example, in a payment system, high security is required while the number of registered users is huge. At present, the one to many Authentication technology providing the authentication accuracy and the authentication speed which can be used in such a system has not been put into practical use.

**Citation List**

**Patent Literatures**

Patent Literature 1: Japanese Patent Application No. 2010-152706
Patent Literature 2: International Publication WO2013/136533
Patent Literature 3: Japanese Patent Application No.2010-191573
Patent Literature 4: International Publication WO2012/014300

**Summary of Invention**

**Technical Problem**

The present invention provides a technique capable of realizing high-speed and highly accurate one to many Authentication without impairing user convenience and system requirement.

**Solution to Problem**

The present invention can be expressed as the inventions described in the following items.

(Item 1)
An individual authentication device comprising,
a query data acquisition unit configured to extract biometric information of human body surface as query surface information and biometric information of inside human body as query inside body information from one color query image obtained by photographing a human body of an individual, and
to generate identification query data from one of the query surface information and the query inside body information, and verification query data from the other of the query surface information and the query inside body information;
an identification unit configured to perform 1:N Identification with the identification query data and a plurality of already-registered identification registration data, and
to identify one or more verification registration data to be used in verification, the one or more verification registration data being associated with the identification registration data; and
a verification unit configured to authenticate a unique individual by performing 1:1 Verification with the identified one or more verification registration data using the degree of similarity with the verification query data; wherein
the identification query data and identification registration data belong to a metric space,
the identification unit configure to identify one or more verification registration data to be used in the verification by performing the 1:N Identification based on the positional relationship between the identification query data and identification registration data in the metric space,
each of the identification registration data has an index value indicating that similar data in the metric space have a same index, and
the identification unit configure to convert the identification query data so that similar data in the metric space has a same index, and
to perform the 1 : N identification with identification registration data and the index generated by the conversion.
(Item 2)
The device of item 1, wherein
the identification query data and the verification query data have same information structure.
(Item 3)
The device of item 1 or item 2, wherein
the 1 : 1 verification is performed on all of the one or more verification registration data.
(Item 4)
The device of any one of item 1 to item 3, wherein
the query surface information and the query inside body information are represented by line components,
the identification query data is data generated by Radon transform performed on one of the query surface information and the query inside body information represented by the line components, and
the verification query data is data generated by Radon transform performed on the other of the query surface information and the query inside body information represented by the line components.
(Item 5)

The device of any one of item 1 to item 4, further comprising:

> a registration unit configured to register the identification registration data associated with the verification registration data,
> to extract biometric information of human body surface as template surface information and biometric information of inside human body as template inside body information from one color template image obtained by photographing a human body of an individual, and
> to generate identification registration data corresponding to the individual from one of the template surface information and the template inside body information, and verification registration data from the other of the template surface information and the template inside body information; wherein
> both the identification registration data and verification registration data have same information structure.

(Item 6)
The device of item5, wherein
the template surface information and the template inside body information are represented by line components,
the identification registration data is data generated by Radon transform performed on one of the template surface information and the template inside body information represented by the line components, and
the verification registration data is data generated by Radon transform performed on the other of the template surface information and the template inside body information represented by the line components.

(Item 7)
The device of any one of item 1 to item 6, wherein
the positional relationship between the identification query data and the identification registration data is a positional relationship between these data and predetermined reference data.

(Item 8)
The device of item 7, wherein
the reference data is plural, and
the identification query data and identification registration data belong to each of groups based on the positional relationship between each reference data and the identification query data or identification registration data, and same group data have same index.

(Item 9)
The device of any one of item 1 to item 8, wherein
the query image is an image of a palm of an authentication target individual, the query surface information is a palm print pattern extracted from the query image, and the query inside body information is a vein pattern extracted from the query image.

(Item 10)
The device of item 5 or item 6, wherein
the template image is an image of a palm of a registration target individual, the template surface information is a palm print pattern extracted from the template image, and the template inside body information is a vein pattern extracted from the template image.

(Item 11)
An individual authentication method comprising:

> in a query data acquisition step, extracting biometric information of human body surface as query surface information and biometric information of inside human body as query inside body information from one color query image obtained by photographing a human body of an individual, and
> generating identification query data from one of the query surface information and the query inside body information, and verification query data from the other of the query surface information and the query inside body information;
> in an identification step, performing 1 : N identification with the identification query data and a plurality of already-registered identification registration data, and identifying one or more verification registration data to be used in verification, the one or more verification registration data being associated with the identification registration data; and
> in a verification step, authenticating a unique individual by performing 1:1 Verification with the identified one or more verification registration data using the degree of similarity with the verification query data; wherein
> the identification query data and identification registration data belong to a metric space,
> in the identification step, identifying one or more verification registration data to be used in the verification by performing the 1:N Identification based on the positional relationship between the identification query data and identification registration data in the metric space,

each of the identification registration data has an index value indicating that similar data in the metric space have a same index, and

in the identification step, converting the identification query data so that similar data in the metric space has a same index, and

performing the 1: N Identification with identification registration data and the index generated by the conversion.

(Item 12)

A computer program for causing a computer to perform each step in item 11.

**Advantageous Effects of Invention**

In the present invention, the query surface information which is the biological information of the human body surface and the query body inside information which is the biological information in the human body are extracted to generate query data having same information structure from one color query image obtained by photographing the biological body of an individual. The identification query data is used to identify the registered identification registration data, and to identify the verification registration data to be used for one or more verifications. Next, 1:1 Verification is performed using the degree of similarity between the verification query data and the verification registration data. As a result, it is possible to provide a technology capable of one to many Authentication for identifying an individual from large-scale registration data at high speed and with high accuracy.

More specifically, the feature of the palm print pattern and vein pattern in the palm of an individual to be authenticated are extracted from one original image data photographed by the image acquisition unit for visible light, and Radon transform is performed to generate identification query data and verification query data. The identification query data can be used to perform identification based on the positional relationship in the metric space with the registered identification registration data. The verification query data can be used to perform 1:1 Verification based on the degree of similarity with the registered verification registration data. This makes it possible to provide one to many Authentication for identifying an individual from large-scale registration data at high speed and with high accuracy.

[Brief description of drawings]

FIG. 1 is a block diagram showing a schematic configuration of an individual authentication device according to an embodiment of the present invention.

Fig. 2 is a block diagram showing a schematic configuration of an authentication image acquisition device.

FIG. 3 is a block diagram showing a schematic configuration of an authentication image processing unit.

Fig. 4 is an explanatory diagram for explaining an example of use of the device acquiring original images for authentication and template.

Fig. 5 is a block diagram showing a schematic configuration of an identification query image processing unit.

Fig. 6 is a block diagram showing a schematic configuration of a verification query image processing unit.

FIG. 7 is a block diagram showing a schematic configuration of a template image acquisition device.

FIG. 8 is a block diagram showing a schematic configuration of a template image processing unit.

Fig. 9 is a block diagram showing a schematic configuration of an identification template image processing unit.

Fig. 10 is a block diagram showing a schematic configuration of a verification template image processing unit.

FIG. 11 is a block diagram showing a schematic configuration of an identification unit.

Fig. 12 is a flowchart showing a schematic flow of an individual authentication method according to an embodiment of the present invention.

FIG. 13 is a flowchart for explaining the process procedure on the template image.

Fig. 14 is an explanatory diagram for explaining an example of an image acquired by processing a template image or an authentication image.

Fig. 15 is a block diagram showing the flow of process on a template image or an authentication image.

FIG. 16 is a flowchart for explaining the process procedure generating the identification table.

FIG. 17 is a flowchart for explaining the process procedure on an authentication image.

FIG. 18 is a schematic block diagram for explaining a first modification of the embodiment as shown in FIG. 1.

**Description of Embodiments**

An embodiment of the present invention will be described below with reference to the attached drawings.

(Configuration of individual authentication system)

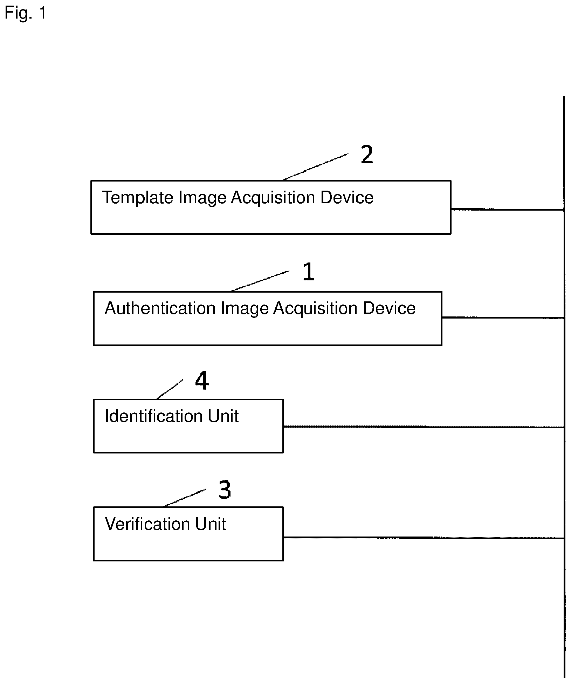

First, the configuration of an individual authentication system according to an embodiment of the present invention will be described based on FIG. 1.

This individual authentication system comprises an authentication image acquisition device 1 (corresponding to an example of a query data acquisition unit) which acquires an authentication image as a query image, a template image acquisition device 2 (corresponding to an example of a registration unit), an identification unit 4, and a verification unit 3 (see FIG. 1). The basic function of the identification unit 4 is to identify (that is, search) a template similar to the authentication image acquired by the authentication image acquisition device 1 and to find the ID corresponding to the template. The basic function of the verification unit 3 is to authenticate a unique individual by verifying the verification image acquired by the authentication image acquisition device 1 with the template corresponding to the ID found by the identification. In this embodiment, mutual functional elements can transmit or receive data via an appropriate network or communication path. The same also applies to the functional elements in each functional block.

(Authentication image acquisition device 1)

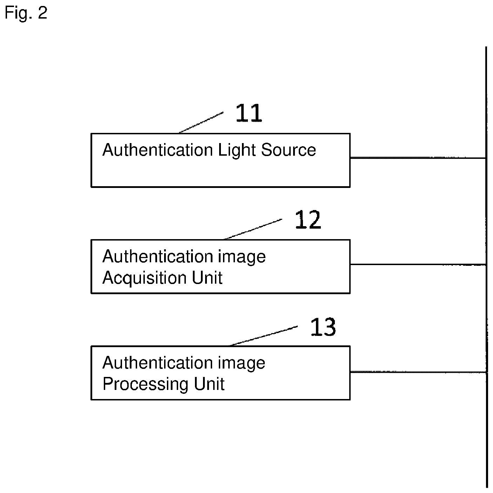

The authentication image acquisition device 1 includes an authentication light source 11, an authentication image acquisition unit 12, and an authentication image processing unit 13 (see FIG. 2).

The authentication light source 11 is configured to be capable of irradiating a palm of a human body with light including at least red light in the visible light region. The authentication light source 11 can be composed of a light-emitting element (for example, an LED) which can emit light with a wavelength in the visible light region including red light. It is basically possible to use sunlight or ambient light as the light source. However, the accuracy of the authentication can be improved by using artificial light as the light source and by accurately grasping the wavelength range of the emitted light. Here, in this specification, red light refers to light having a wavelength of approximately 580 to 750 nm (nanometers), so-called redish light, but the optimum wavelength can be determined experimentally. Amber Light (wavelength of approximately 590 to 630 nm) is considered to be more preferable. Further, the light source may emit only light in these wavelength bands, but may include light of other wavelengths. Further, A light source which emits desired light by filtering can also be used. However, visible light other than red light may act as noise in the extraction of vein pattern. Therefore, in order to reduce noise, a light source emitting only red light is preferable.

The authentication image acquisition unit 12 is configured to acquire at least one reflection image (that is, image data) composed of light emitted from the authentication light source 11 and reflected by the palm of the human body. The authentication image acquisition unit 12 as described above can be configured by an appropriate device such as a digital camera or an image scanner. Alternatively, the authentication image acquisition unit 12 can be configured by a camera attached to the mobile device.

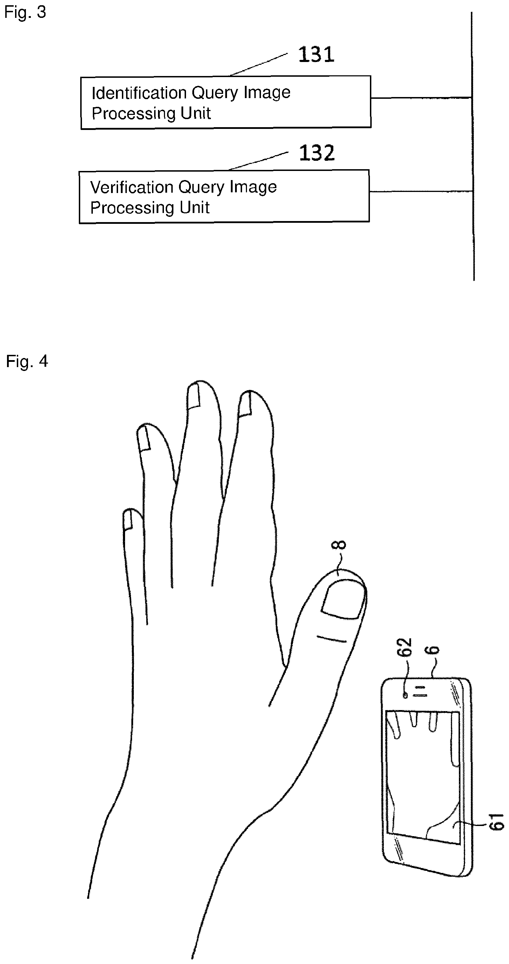

The authentication image processing unit 13 includes an identification query image processing unit 131 and a verification query image processing unit 132 (see FIG. 3). The identification query image processing unit 131 performs image process on the reflection image to extract a palm print pattern of the palm for identification from one reflection image. The identification query image processing unit 131 is configured to extract the palm print pattern by converting the data corresponding to the reflection image of the palm into a grayscale value based on the R signal, G signal, and B signal in the RGB color space.

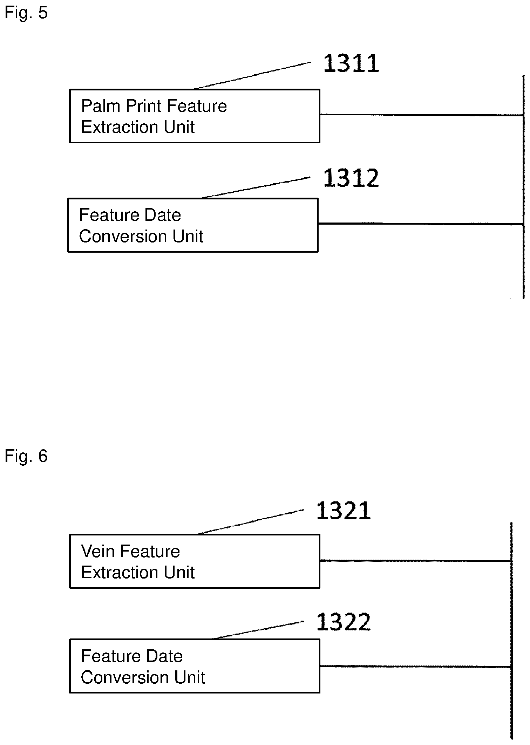

The identification query image processing unit 131 includes a palm print feature extraction unit 1311 and a feature data conversion unit 1312 (see FIG. 5). The palm print feature extraction unit 1311 performs the conversion to the gray scale value described above as the operation of the identification query image processing unit 131, and extracts the palm print pattern as a binary image. The feature data conversion unit 1312 performs Radon transform on the binary image, extracts the frequency spectrum in the radial direction from the transformed image, and then converts the frequency spectrum into the phase-only image transformed in the logarithmic polar coordinate system to generate identification feature data (corresponding to identification query data).

The verification query image processing unit 132 converts the data corresponding to the reflection image of the palm into the HSV color space, changes the phase of the H signal and the intensity of the S signal in the HSV color space, and then converts the HSV color space into an RGB color space and a CMYK color space to extract a vein pattern as a obtained color signal. Details of this image process will be described later.

The verification query image processing unit 132 includes a vein feature extraction unit 1321 and a feature data conversion unit 1322 (see FIG. 6). The vein feature extraction unit 1321 extracts the color signal described above as the operation of the verification query image processing unit 132, and further extracts the extracted vein pattern as a binary image. The feature data conversion unit 1322 performs Radon transform on the binary image, extracts the frequency spectrum in the radial direction from the transformed image, and then converts the frequency spectrum into the phase-only image transformed in the logarithmic polar coordinate system to generate verification feature data (corresponding to verification query data). Since the feature data conversion unit 1322 performs the same process as the feature data conversion unit 1312 of the identification query image processing unit 131, both can be implemented as same computer or computer program.

Here, since the identification query data and the verification query data in this embodiment are generated by the same process, they have same information structure. The same information structure means a data structure composed of information series having the same semantic scale. For example, both the binary images for the identification and verification have position information of the line segments of the palm print and the vein, and these have the same information structure. In addition to the process described above, it can be said that information sequences generated by extracting local feature amount and global feature amount from images in which palm prints and veins are emphasized, and the images from which only phase components are extracted, have the same information structure. Having the same information structure means having same amount of information, and thus having same identification accuracy if same threshold value is set. Here, the same amount of information means that they have the same semantic scale and the same data dimension, that is, data length. The semantic scale here means the meaning of information and the standard of its numerical value. Each of the local feature amount, the global feature amount, the phase component and the position information of the line segment, described above and the like, has feature information and is semantic scale having a standardized numerical value.

Both the authentication light source 11 and the authentication image acquisition unit 12 can be implemented on one mobile terminal. An example of such an implementation is shown in FIG. 4. Here, a smartphone is used as the mobile device 6, and reference numeral of 8 in the figure indicates a human hand.

The mobile device 6 comprises a display 61 capable of emitting light including red light to the outside, and an attached camera 62. Then, in the example of FIG. 4, the authentication light source is configured by the display 61, and the authentication image acquisition unit is configured by the camera 62. In addition, the mobile device 6 in FIG. 4 includes a backlight (not shown) for illuminating the display 61, and the light from the backlight can illuminate the display 61 by passing through it. Generally, the display of a smartphone is often composed of a liquid crystal display. The liquid crystal panel can adjust the transmission color and the transmission amount of the light from the backlight by controlling the color filter or the polarization filter. Therefore, by controlling the liquid crystal panel to generate light in the wavelength range suitable for this embodiment, the operation as the light source of this embodiment becomes possible. As the display, instead of the liquid crystal display, it is possible to use an appropriate display such as an organic EL display or an LED display which can emit light of a required wavelength.

(Template image acquisition device)

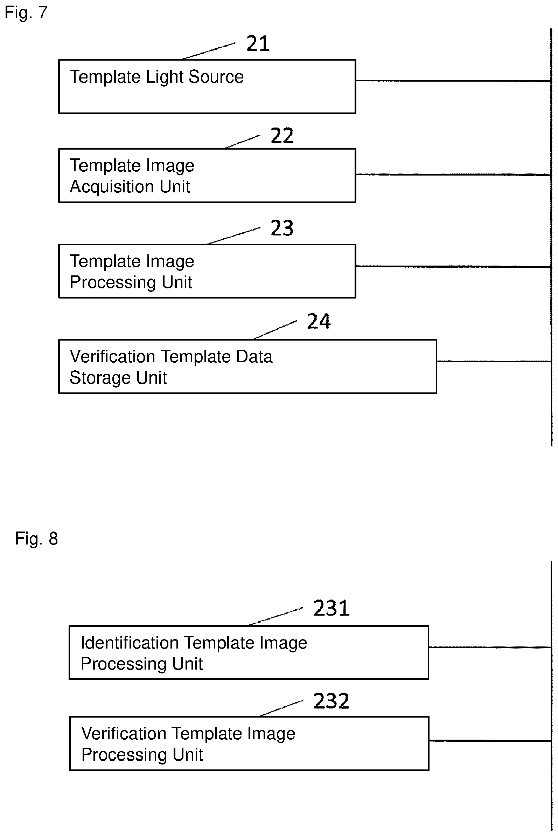

The template image acquisition device 2 (corresponding to a registration unit) includes a template light source 21, a template image acquisition unit 22, a template image processing unit 23, and a verification template data storage unit 24 (see FIG. 7). The template data means registered data.

The template light source 21 is configured to be capable of irradiating a palm of the human body with light including at least red light in the visible light region. The template light source 21 can be configured similarly to the authentication light source 11 described above. It is also possible to use one light source for both purposes.

The template image acquisition unit 22 is configured to acquire, from the template light source 21, at least one reflection image composed of light reflected by the palm of the human body. The template image acquisition unit 22 can be configured similarly to the authentication image acquisition unit 12 described above, and one image acquisition unit (for example, a camera) can be used for both purposes.

The template image processing unit 23 includes an identification template image processing unit 231 and a verification template image processing unit 232, and performs image process on the reflection image to generate an identification template and a verification template from one reflection image (see FIG. 8). The identification template image processing unit 231 performs the same conversion to the grayscale value as the process by the identification query image processing unit 131 described above, and generates the template data by extracting print pattern of a palm for template.

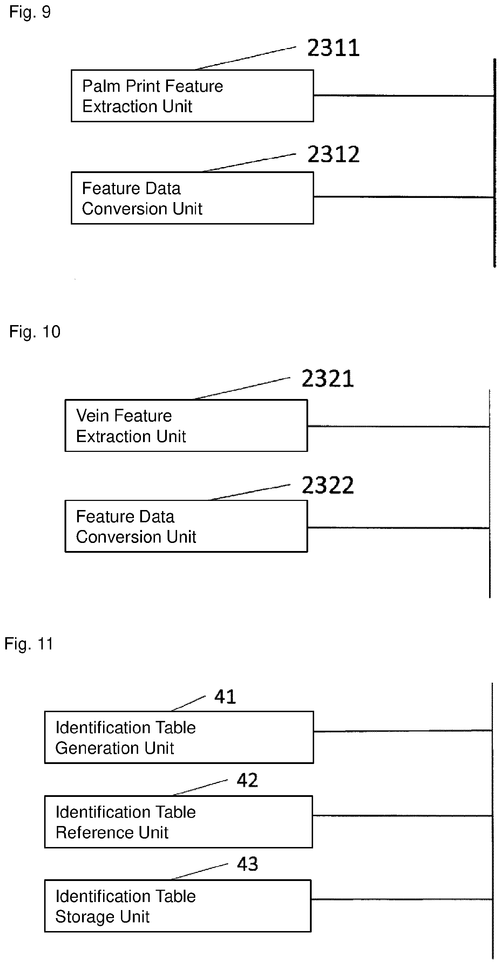

The identification template image processing unit 231 includes a palm print feature extraction unit 2311 and a feature data conversion unit 2312 (see FIG. 9). The palm print feature extraction unit 2311 performs the above-described conversion to the grayscale value as a function of the identification template image processing unit 231, and extracts a palm print pattern as a binary image. The feature data conversion unit 1322 performs Radon transform on the binary image, extracts the frequency spectrum in the radial direction, and then converts into the phase-only image transformed in the logarithmic polar coordinate system to generate identification feature data (corresponding to identification template data). Since the identification template image processing unit 231 performs the same process as the identification query image processing unit 131, both can be implemented as same computer or computer program.

The verification template image processing unit 232 performs the same conversion to the color signal as the process of the verification query image processing unit 132 described above, and generates the verification template data by extracting the vein pattern in the palm for template.

The verification template image processing unit 232 includes a vein feature extraction unit 2321 and a feature data conversion unit 2322 (see FIG. 10). The vein feature extraction unit 2321 performs the conversion to the color signal described above and extracts the vein pattern as a binary image. The feature data conversion unit 2322 performs Radon transform on the binary image, extracts the frequency spectrum in the radial direction, and then converts into the phase-only image transformed in the logarithmic polar coordinate system to generate verification feature data (corresponding to verification template data). Since the verification template image processing unit 232 performs the same process as the verification query image processing unit 132, both can be implemented as the same computer or computer program.

Here, since the identification registration data and the verification registration data in the present embodiment are generated by the same process, they have same information structure. The same information structure means a data structure composed of information series having the same semantic scale. Specifically, the above-described phase-only images for identification and verification are both phase images and have the same semantic scale. The phase image is an image expressing the frequency spectrum in the radial direction in a logarithmic polar coordinate system. The frequency spectrum in the radial direction is obtained by Radon-transform on the binary image. Here, process other than Radon transform is also possible. For example, all the images generated by the above-mentioned binarization have position information of the line segments of the palm print and the vein, and these have the same information structure. Furthermore, process other than these is also possible, and it can be said that information sequences generated by extracting local feature amount and global feature amount from images in which palm prints and veins are emphasized, and the images from which only phase components is extracted, have the same information structure. Having the same information structure means having the same amount of information, and thus having the same identification accuracy. Here, the same amount of information means that they have the same semantic scale and the same data dimension, that is, data length.

The verification template data storage unit 24 is configured to store the verification template data generated by the verification template image processing unit 232. The verification template data storage unit 24 can be configured by a memory for a computer, for example. Further, the verification template data storage unit 24 can be configured by an appropriate device capable of recording digital data, such as a hard disk, an optical disk, a magneto-optical disk, or a semiconductor memory.

(Identification unit)

The identification unit 4 includes an identification table generation unit 41, an identification table reference unit 42, and an identification table storage unit 43 (see FIG. 11).

The identification table generation unit 41 generates an index value based on the identification template data such that similar data has the same table index. The conversion to the index value is performed such that the closer the positions of the images representing the identification feature data in the Euclidean metric space are, the more the index values are similar. The process contents of the conversion to the index value will be described later.

The identification table storage unit 43 stores an ID corresponding to an individual in a table (specifically, an index) in association with the index value converted based on the identification template data.

The identification table reference unit 42 converts the identification feature data (identification query data) acquired by the authentication image acquisition device 1 into an index value. The conversion is performed such that the closer the positions of the images representing the identification feature data in the Euclidean metric space are, the more the index values are similar. Then, the identification table reference unit 42 references the identification table storage unit 43 to acquire the ID stored in the index value. The contents of the identification process will be described later.

The acquired ID corresponds to the ID of the identification template data similar to the identification data. Here, multiple ID may be acquired.

The verification unit 3 is configured to authenticate an individual by verifying the verification data (verification query data) acquired by the authentication image acquisition device 1 and the template data (verification registration data) stored in the verification template data storage unit 24. The template data corresponds to the ID acquired by the identification unit 4. The contents of the verification process will be described later.

(Procedure of individual authentication)

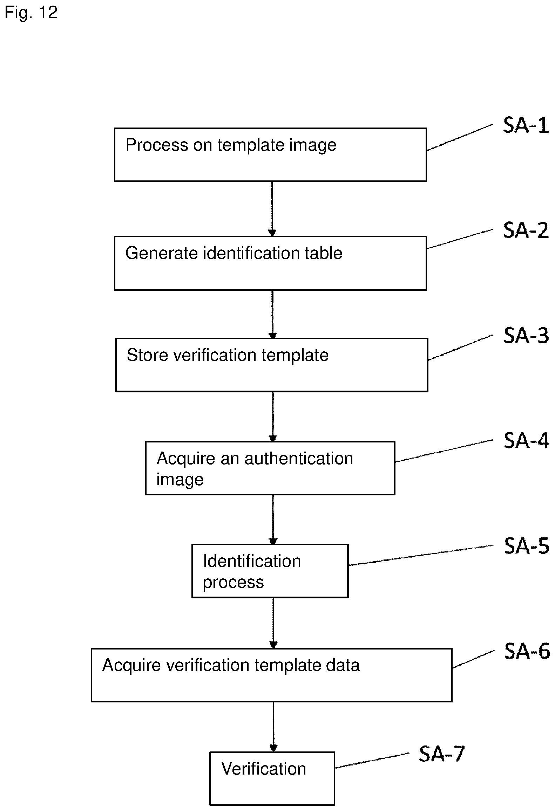

Next, individual authentication method using the aforementioned individual authentication system will be described with reference to FIG. 12 to FIG. 17.

(The entire procedure)

The entire flow of individual authentication according to this embodiment is shown in FIG. 12.

(SA-1 in Fig. 12)

First, the template image processing unit 23 generates an identification template data and the verification template data from one reflection image obtained by photographing a palm of a user.

(SA-2 in Fig. 12)

Next, based on the generated identification template data, generating a table index value is performed, and the user ID is stored in the relevant index of the identification table. Here, the table index value corresponds to the grouping number of the identification template data to be registered, and the conversion to the table index value means obtaining the group number at the grouping, and the template data having the same table index value belongs to the same group. This process is performed by the identification table generation unit 41.

For storing ID, in addition to storing in the normal direct address table, it is also possible to store in a chain method table or in a tree structure table. Since the chain method and the tree structure are widely known, detail description is omitted here.

(SA-3 in Fig. 12)

Next, the generated verification template data is stored in the verification template data storage unit 24 in association with the ID.

(SA-4 in Fig. 12)

Next, at the authentication, an authentication image (one color reflection image) is obtained by photographing a palm of a user. Then, using this authentication image, identification data (identification query data) and verification data (verification query data) are generated.

(SA-5 in Fig. 12)

Then, the identification table reference unit 42 performs conversion to the table index value based on the identification data, refers to the identification table, and acquires the ID stored in the index.

(SA-6 in Fig. 12)

Next, the verification unit 3 acquires the verification template data corresponding to the acquired ID from the verification template data storage unit 24.

(SA-7 in Fig. 12)

Next, the verification unit 3 performs an individual authentication by performing 1:1 Verification on the acquired verification template data with the verification data using the degree of similarity.

Details of each process described above will be explained in further detail below.

(Process of template image)

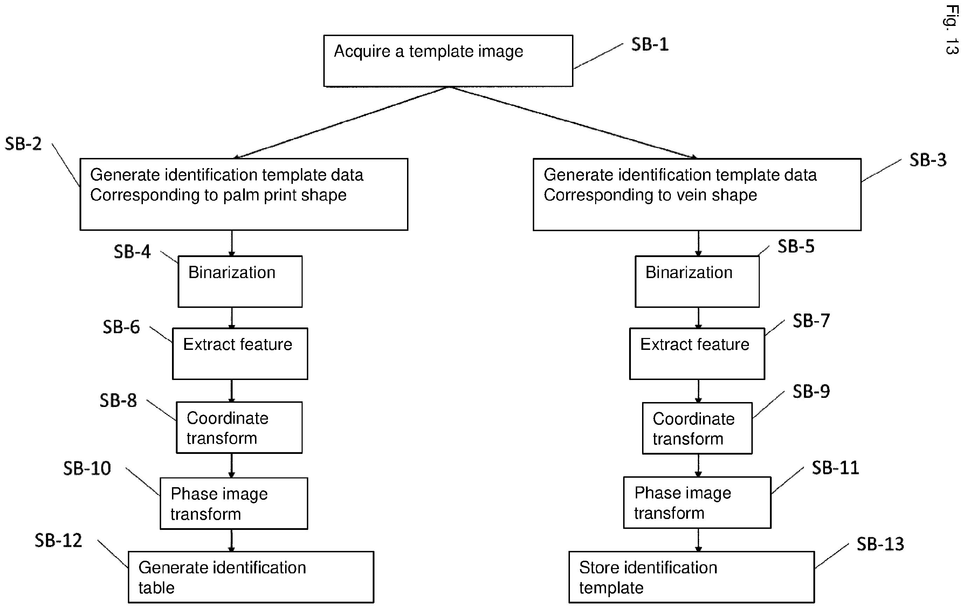

(Step SB-1 in FIG. 13)

Before the authentication process, the template image is processed according to the following procedure. First, a palm of a human body is irradiated with light including at least red light in the visible light region from the template light source 21. Then, the template image acquisition unit 22 acquires at least one reflection image composed of the light reflected by the palm of the human body. Here, as the color space of the image acquired by the template image acquisition unit 22 in a hardware-like manner, RGB is generally represented by true color of 256 gradations, but it is also possible to use other formats. Rather, many common devices (for example, cameras) acquire data in the YUV color space in a hardware-like manner. In this case, for example, the data in the YUV color space can be converted by software to generate the data in the RGB color space, which can be used for the subsequent calculation. Needless to say, the template image acquisition unit 22 may be configured to acquire the RGB color space data in a hardware. Here, it should be noted that the RGB color space and the YUV color space have a complementary color relationship which allows mutual conversion.

(Step SB-2 in FIG. 13)

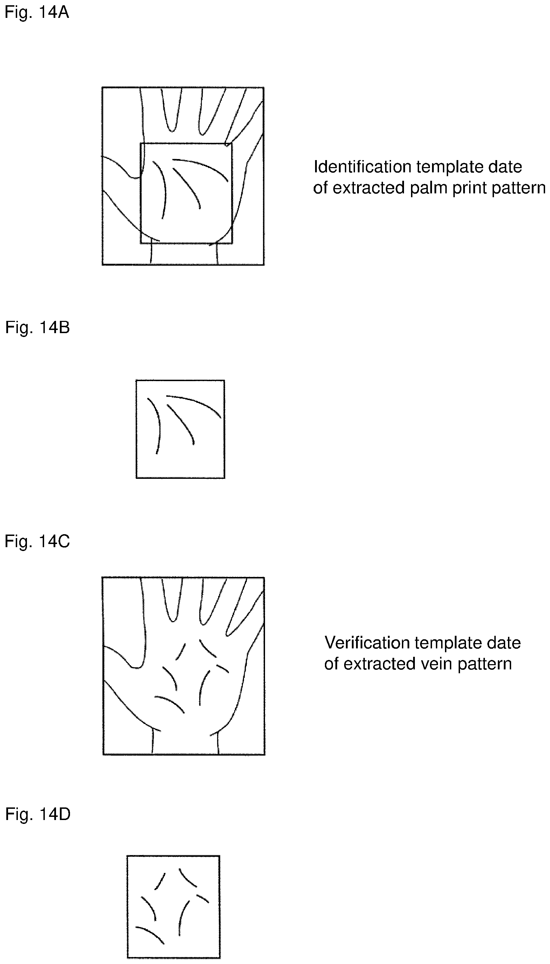

Then, the palm print feature extraction unit 2311 of the identification template image processing unit 231 performs image process on the reflection image to extract a template palm print pattern of the palm from the one reflection image as an identification template data (corresponding to a template surface information) to generate identification registration data (see FIG. 14A). As the identification template data, the pattern data as shown in FIG. 14A can be abstracted to form the pattern data of only the palm print portion as shown in FIG. 14B.

The palm print feature extraction unit 2311 of the identification template image processing unit 231 converts the RGB color space data acquired by the template image acquisition unit 22 to generate, for example, a bitmap image, and further performs conversion to a grayscale image to extract the features of the palm print pattern. Note that the palm print is a print represented by minute irregularity on the palm, and has a characteristic pattern which differs depending on each individual.

Known methods can be used to extract the palm print pattern. For example, an edge image representing a palm print can be generated from the original image by applying gray scale conversion and a Laplacian filter.

In the present embodiment, the low-pass filter process is performed on the original image, and the processed image is edge-enhanced by the gabor filter to generate a grayscale in order to acquire the pattern features of the palm print, in particular, with the palm line emphasized. It is preferable to perform erosion process additionally on the generated grayscale image to generate identification template data in which the palm print pattern, especially the palm line, is emphasized. Here, the low-pass filter, the gabor filter, and the erosion process are widely known, detailed description is omitted.

(Step SB-3 in FIG. 13)

In parallel with, before, or after these process, the vein feature extraction unit 2321 of the verification template image processing unit 232 performs image process on the reflection image to extract a template vein pattern in the palm from one reflection image as verification template data (corresponding to template inside body information) (see FIG. 14C). As the verification template data, the pattern as shown in FIG. 14C can be abstracted to form pattern data of only the vein portion as shown in FIG. 14D. The vein pattern extraction process will be described in detail below.

It is necessary to find information which represents the vein pattern strongly and to extract the feature from the original image acquired by the template image acquisition unit 22. Here, according to the knowledge of the present inventor, in the image acquired by irradiating the palm with red light, vein pattern appears most strongly in the M (magenta) signal of the CMYK color space. And it is in the G signal in the RGB color space that the vein pattern does not appear and the palm print pattern is displayed.

Furthermore, in addition to these two-color signals, the R signal in the RGB color space in which both vein pattern and palm print pattern tend to appear is added, and the process described below is performed to generate verification template data.

First, the RGB values of each pixel on the original image are HSV converted and mapped on the hue circle. Next, the R signal value, the G signal value, and the B signal value (that is, the phase of the hue H in the HSV space) mapped on the hue circle are shifted by appropriately set values. Further, the intensity (magnitude) of the saturation (value of S) in the HSV space is changed to an appropriately set value. The amount of this change can be determined experimentally.

In order to convert the image data in the RGB color space into the HSV space, the following formula can be generally used.

$$H = 60 * (G\text{-}B) / (MAX [R, G, B] \text{-}MIN [R, G, B]) \text{ if } R = MAX [R, G, B]$$

$$H = 60 * (B\text{-}R) / (MAX [R, G, B] \text{-}MIN [R, G, B]) + 120 \text{ if } G = MAX [R, G, B]$$

$$H = 60 * (R\text{-}G) / (MAX [R, G, B] \text{-}MIN [R, G, B]) + 240 \text{ if } B = MAX [R, G, B]$$

$$S = MAX [R, G, B] \text{-}MIN [R, G, B]$$

$$V = MAX [R, G, B]$$

In the present embodiment, the R signal and the G signal in the RGB color space are changed to the R' signal and the G' signal generated by reducing the saturation (value of S) by 30% along the negative direction in the HSV space. Further, the M (magenta) signal in the CMYK color space is changed to the M' signal generated by shifting the phase of H by + 15 ° in the HSV space and further reducing the value of S by 30% along the negative direction. The width of shift in hue (that is, width of change) and the value of change in saturation are determined by experiment.

By the above process, data in the R' signal, G' signal, and M' signal, which is different from the original RGB space and CMYK space data, can be acquired. In the present embodiment, the R', G', and M' space data obtained as above can be represented as 8-bit (256 gradations) grayscale images.

$$GPvein = (\alpha 1 * R' + \alpha 2 * M' - \alpha 3 * G')$$

Here,

GPvein : Grayscale data obtained from the values of R' signal, G' signal and M' signal.

R' : A value obtained by converting the R signal value in the RGB color space into the HSV color system, changing the saturation (-30%), and returning to the RGB color system.

G': A value obtained by converting the G signal value in the RGB color space into the HSV color system, changing the saturation (-30%), and returning to the RGB color system.

M' : A value obtained by converting the magenta signal value in the CMYK color space into the HSV color system, changing the hue (+ 15 °) and the saturation (-30%), and returning to the CMYK color system.

$\alpha 1, \alpha 2,$ or $\alpha 3$ : coefficient (determined experimentally).

For example, the optimum coefficient values determined experimentally are

$$GPvein = (0.6 * R' + 0.6 * M' - 0.2 * G')$$

Here, the calculation of GPvein is performed by each pixel. If the calculation result by each pixel is 0 or less, the value of GPvein is set to 0, and the result is 255 or more, the value of GPvein is set to 255. In this way, the verification template data can be generated as a grayscale image in which the vein pattern is emphasized.

However, the above formula for obtaining GPvein is just an example, and the specific formula is not limited. The specific calculation formula can be appropriately set by an experiment which can be performed by those skilled in the art based on the above-mentioned knowledge. The formula need not be represented by a linear combination.

In the above, the example using the R signal and the G signal in the RGB color space and the magenta signal in the CMYK color space have been described, but the B signal in the RGB color space, and the cyan signal and the yellow signal in the CMYK color space can be used additionally.

Furthermore, in the above, the RGB color space and the CMYK color space are directly used, but a color space which can be converted into the RGB color space (for example, YCbCr, YIQ, Luv, Lab, XYZ) can be used instead of the RGB color space to extract feature data in a template image or a query image. That is, the data in the RGB space and the data in the color space convertible with the RGB space can be converted by a predetermined formula. Therefore, the above description also applies to the case where data other than in the RGB color space is used by interposing a predetermined data conversion. That is, it is within the scope of the invention, instead of the data representing the feature in the RGB space in the present invention, to represent the feature of the image by using the data obtained by mapping in another color space or to perform the identification using a feature quantity represented in this way.

The optimum value can be experimentally determined for each coefficient in the above description. The coefficient may be a negative value. Further, the coefficient $\alpha$ is generally experimentally determined by an external light source environment (for example, brightness).

(Steps SB-4 and SB-5 in FIG. 13)

Next, the palm print feature extraction unit 2311 of the identification template image processing unit 231 and the vein feature extraction unit 2321 of the verification template image processing unit 232 binarize the grayscale template data (both for identification and verification), respectively.

Since the template data (TD) can be binarized by a general method such as taking a moving average at each pixel or each block, a detailed description is omitted here. The binarization is performed on both the identification template data and the verification template data, but the each binarization method is not necessarily same. Binarization is to extract line segment pattern information of a palm print and vein, and the extracted data has position information of each line segment of the palm print and vein. Therefore, even if the binarization methods for these data are different, it can be said that they have same information structure.

(Steps SB-6 and SB-7 in FIG. 13)

Next, the feature data conversion unit 2312 of the identification template image processing unit 231 and the feature data conversion unit 2322 of the verification template image processing unit 232 perform feature extraction of template data (both for identification and verification). Radon transform is used as a method for extracting the feature. In this method, a template image which is two-dimensional, is projected on the axis in the $\theta$ direction ($\theta$ = 0 to 180 °) and is expressed by a position p on the projection axis and an angle $\theta$.

(Steps SB-8 and SB-9 in FIG. 13)

Next, the feature data conversion unit 2312 of the identification template image processing unit 231 and the feature data conversion unit 2322 of the verification template image processing unit 232 perform Fourier transform in the p direction on the Radon-transformed feature data, and extract only the amplitude component. Specifically, the amplitude component is obtained by taking the square root of the sum of squares of the real part and the imaginary part after the Fourier transform. By extracting only amplitude component, the extracted feature data becomes linear shift-invariant in the p direction.

Next, the feature data conversion unit 2312 of the identification template image processing unit 231 and the feature data conversion unit 2322 of the verification template image processing unit 232 perform logarithmic coordinate conversion in the p direction. Specifically, p is converted to log (p), and the feature data is in a logarithmic polar coordinate system.

(Steps SB-10 and SB-11 in FIG. 13)

Next, the feature data conversion unit 2312 of the identification template image processing unit 231 and the feature data conversion unit 2322 of the verification template image processing unit 232 perform the conversion on the feature data in the logarithmic polar coordinate system into a phase-only image in which only the phase is extracted, in order to make the following calculation process easy. Specifically, two-dimensional feature data in a logarithmic polar coordinate system is subjected to two-dimensional Fourier transform, the amplitude component is set to 1, and then two-dimensional inverse Fourier transform is performed.

Since phase-only image conversion is also well known, detailed explanations are omitted. In this embodiment, the phase-only image converted data is data representing the characteristics of the template images for both identification and verification (that is, registration data for identification and verification).

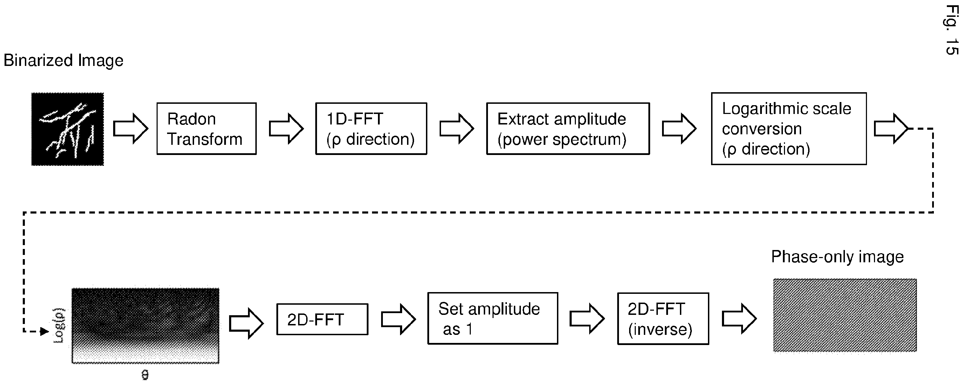

FIG. 15 shows a detailed process flow in which data is converted from binary image to phase-only image conversion data. The contents of each process are as described above.

(Step SB-12 in FIG. 13)

Next, the template image processing unit 23 passes the identification template data (identification registration data) on which the above process is performed and the ID number corresponding to the individual to the identification table generation unit 41 of the identification unit 4. At the same time, the verification template data (verification registration data) is stored in the verification template data storage unit 24 in association with the ID number corresponding to the individual. The above-mentioned process is usually performed before identification. The identification process will be described later.

(Identification table generation process)

A specific example of identification table generation process will be described below.

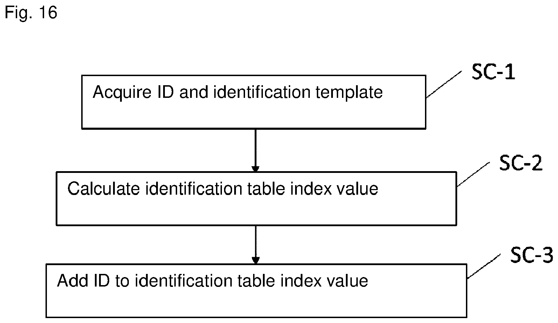

(Step SC-1 in FIG. 16)

The identification table generation unit 41 acquires the ID of the target individual and identification template data (identification registration data).

(Step SC-2 in FIG. 16)

Next, the identification table generation unit 41 performs conversion based on the identification template data (identification registration data) so that similar data have same index value. Specifically, the identification template data is considered to be a template vector having a dimension of the data length, and is converted into an index value so that vectors being close to each other in the Euclidean metric space have the same table index.

One of the methods to realize that vectors being close to each other in Euclidean metric space have the same table index is Locality-sensitive Hashing (LSH). According to this, by applying a random hash function to the template vector, it is possible to realize that close in the Euclidean distance, that is, similar vector has the same hash value.

Also, it is possible to perform clustering based on Euclidean distance so that data in a same class at the clustering have the same index.

Furthermore, the above-mentioned distance is not limited to a direct distance between two data, but may be each distance with respect to some reference data.

It is also possible to set a plurality of reference vectors and group template vectors (that is, data) based on the positional relationship between each reference vector and the template vector, for example, the nearest neighbor reference vector, the distance scale relationship between each reference vector, or the order of the distances, so that data in the same group have the same index. By using this method, it is also easy to generate an index based on whether "the distance between the template vector and the reference vector is within (true pattern) or without (false pattern) a certain range" (referred as true-false pattern). Furthermore, it is possible to obtain an index as an output of these distance calculation, or machine learning or deep learning of the true-false pattern. Since there are various generally known methods for machine learning and deep learning, the description thereof will be omitted here.

Here, in addition to Euclidean distance, any distance function such as Manhattan distance, Chebychev distance can be applied.

Since these distances can be calculated by a general method, detailed description thereof will be omitted here.

(Step SC-3 in FIG. 16)

Next, the identification table generation unit 41 refers to the identification table stored in the identification table storage unit 43, and additionally stores the ID of the target individual in the table index corresponding to the converted index value. If the corresponding index does not exist in the table, the ID is additionally stored after adding the index to the table.

(Process of authentication image)

The process of the authentication image will be described below. The process of the authentication image can be basically performed in the same way as the process of the template image.

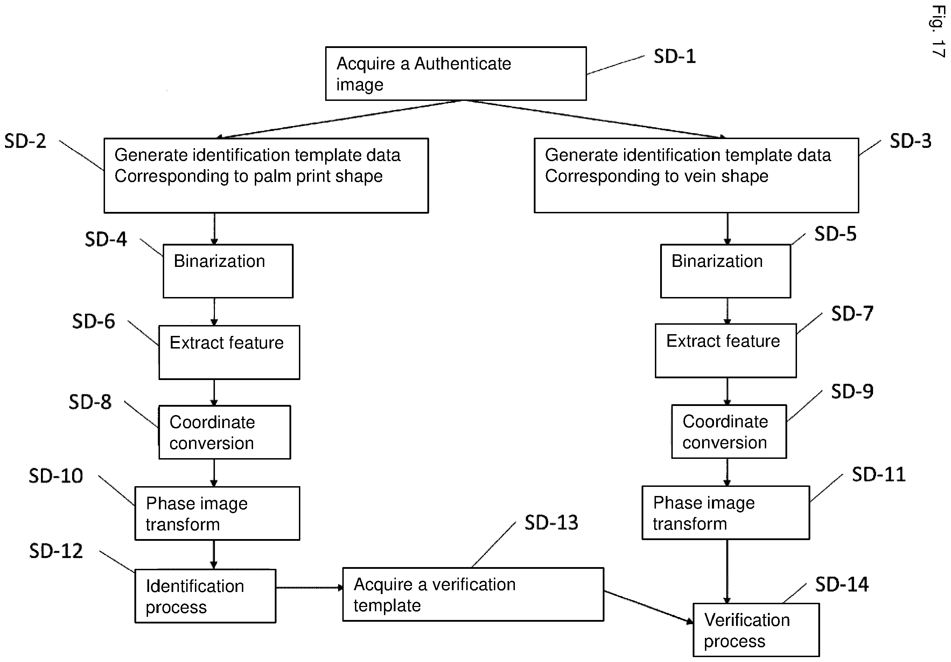

(Step SD-1 in Fig. 17)

First, the authentication light source 11 irradiates a palm of a human body with light including at least red light in the visible light region. Then, the authentication image acquisition unit 12 acquires at least one reflection image composed of the light reflected by the palm of the human body.

(Step SD-2 in Fig. 17)

Next, the identification query image processing unit 131 of the authentication image processing unit 13 performs image process on the reflection image to extract the palm print pattern of the palm from one reflection image as identification data (query surface information) (see FIG. 14A). Note that FIG. 14 is an explanatory diagram of template data, but since the same process is performed for query data, FIG. 14 is referred here. As the identification data, the pattern as shown in FIG. 14A can be abstracted to form pattern data of only the palm print portion as shown in FIG. 14B. Here, whether performing the abstraction or not is determined according to the process at generating the template data.

(Step SD-3 in FIG. 17)

In parallel with, before, or after this, the verification query image processing unit 132 of the authentication image processing unit 13 extracts the vein pattern in the palm from one reflection image as verification data (query inside body information) by performing the image process on the reflection image (see FIG. 14C). As the verification data, the pattern as shown in FIG. 14C can be abstracted to form pattern data of only the vein portion as shown in FIG. 14D.

(Steps SD-4 and SD-5 in FIG. 17)

Next, the palm print feature extraction unit 1311 of the identification query image processing unit 131 and the vein feature extraction unit 1321 of the verification query image processing unit 132 binarize the grayscale identification data and the verification data.

(Steps SD-6 and SD-7 in FIG. 17)

Next, the feature data conversion unit 1312 of the identification query image processing unit 131 and the feature data conversion unit 1322 of the verification query image processing unit 132 perform the feature extraction of the identification data and the verification data. The feature extraction is the same as the template image process described above, that is, the Radon transform is performed and then the amplitude component of the Fourier transform is extracted.

(Steps SD-8 and SD-9 in FIG. 17)

Next, the feature data conversion unit 1312 of the identification query image processing unit 131 and the feature data conversion unit 1322 of the verification query image processing unit 132 perform coordinate conversion of the feature extracted data. The coordinate conversion is the same as the template image process described above, and is conversion to the logarithmic polar coordinate system.

(Steps SD-10 and SD-11 in FIG. 17)

Next, the identification data and the verification data performed on the process described above converted into a phase-only image as identification query data and verification query data, in order to make the following calculation process easy, in the same way as the template image process described above.

(Step SD-12 in FIG. 17)

Next, the identification table reference unit 42 of the identification unit 4 performs the same conversion as the identification table generation process (SB-12) based on the identification query data to generate index value such that similar data have same index value. Next, the identification table reference unit 42 refers to the identification table stored in the identification table storage unit 43 to acquire ID stored in a table index corresponding to the index value generated by the conversion.
At this time, there may be a plurality of IDs stored corresponding to the index value. Here, the identification table reference unit 42 can refer not only to the same index value as the index value generated by the conversion, but also to the index values in the vicinity thereof. Such a method is known as, for example, Multi-probe LSH. Other methods can be used for referring to the index value in the vicinity. As a method for referring to the index value in the vicinity, various already known methods can be used, and a detailed description thereof is omitted.

(Step SD-13 in FIG. 17)

Next, the verification template data corresponding to the acquired ID is acquired from the verification template data storage unit 24. If there are a plurality of identified IDs, all verification template data corresponding to each ID is acquired.

(Step SD-14 in FIG. 17)

Next, the verification unit 3 performs 1:1 Verification on the acquired verification template data (verification registration data) corresponding to the ID with the verification query data generated by the process of SD-11. The degree of similarity at the verification can be determined by using the maximum value or a value obtained from the vicinity thereof as a scale of a correlation between the verification template data and the verification query data. Then, the identity of an individual can be determined by a predetermined threshold value. In addition, the rotation angle ($\theta$) and the magnification ($p$) can be calculated from the position on the image where the maximum correlation value occurs. When a plurality of IDs is identified, the ID having the highest correlation among them can be selected to perform accurate individual authentication.

According to the present embodiment, the feature of the vein pattern and the palm print pattern in the palm of an individual to be identified can be extracted from one original image data photographed by using the image acquisition unit for visible light (for example, the camera for visible light). Thus, it becomes possible to easily perform individual authentication with high accuracy. As a result, it also becomes possible to simplify, reduce the weight, and reduce the cost of the device.

Moreover, in the present embodiment, the palm print extraction and the vein pattern extraction can be performed by using one light source (one which emits red light), and in this respect, the device can be simplified, the weight and cost can be reduced. However, it is also possible to use a plurality of light sources in the present invention.

(Modification 1)

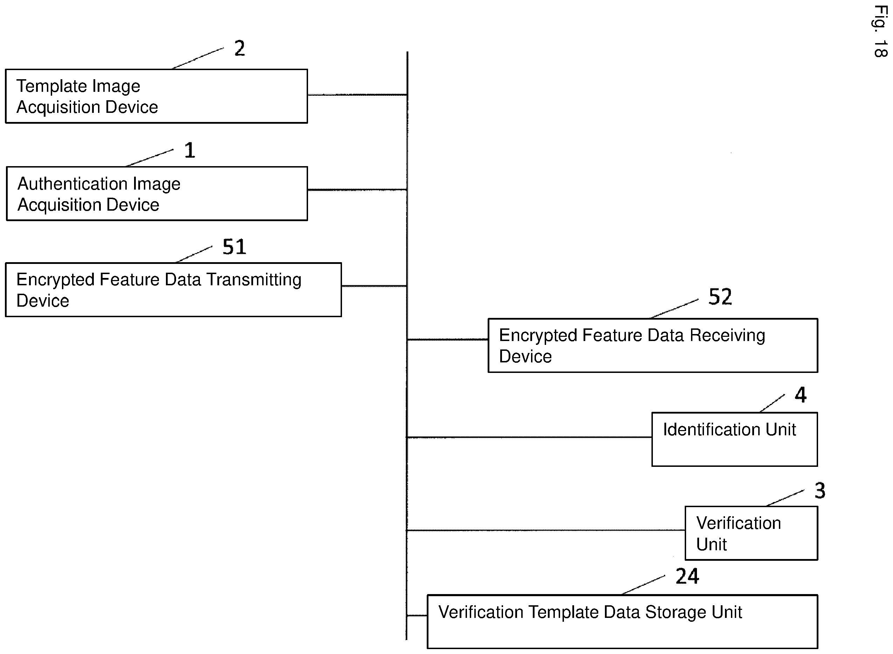

In the above embodiment, the authentication system is configured to include the authentication image acquisition device, the template image acquisition device, the identification unit, and the verification unit. On the contrary, the system of the first modification is configured to further include the encrypted feature data transmitting device 51 and the encrypted feature data receiving device 52 (see FIG. 18). In the following example, the authentication image acquisition device 1, the template image acquisition device 2 which does not include the verification template data storage unit 24, and the encrypted feature data transmitting device 51 are mounted on a mobile device such as a smartphone. Further, the identification unit 4, the verification unit 3, the verification template data storage unit 24, and the encrypted feature data receiving device 52 are mounted on a server. This configuration communicates each other via a network. Of course, such a system configuration is only an example, and other configurations are possible.

In the system of the first modification, specifically, it is possible to realize a system which enables credit card payment by performing individual identification on the server using the smartphone. For example, it can be applied to online shopping or card-less payment at stores.

The template data and the query data acquired by the template image acquisition device 2 and the authentication image acquisition device 1 are transmitted to the server after being encrypted into encrypted feature data by the encrypted feature data transmitting device 51. Since the feature data may be stolen or decrypted to be misused such as spoofing, the feature data is encrypted before transmitting.

The encrypted feature data receiving device 52 acquires and decrypts the encrypted template data and query data. When the identification template data is received, the identification table generation unit 41 of the identification unit 4 generates an identification table and stores it in the identification table storage unit 43. When the verification template data is received, the verification template data storage unit 24 stores the verification template data.

When the encrypted feature data receiving device 52 receives the identification query data and the verification query data, identification table reference unit 42 of the identification unit 4 refers to the identification table, and acquires the ID corresponding to the same index as the index value of the identification query data. Then, the verification unit 3 verifies the verification template data of the target ID with the received verification query data. If the verification is match, the individual authentication is successful.

The method of this embodiment can be implemented by a computer program which can be executed by a computer. Further, this program can be recorded in various type computer-readable medium.

It should be noted that the scope of the present invention is not limited to the above embodiment, and various modifications can be made without departing from the gist of the present invention.

For example, each component described above may exist as a functional block, and need not to exist as independent hardware. As a mounting method, hardware or computer software may be used. Furthermore, one functional element in the present invention may be realized by a set of a plurality of functional elements. Also, a plurality of functional elements in the present invention may be realized by one functional element.

In addition, the query surface information and the query inside body information are not limited to palm prints and palm veins. It is sufficient to be extracted from one color query image such as, face and iris patterns, face and face veins, eye shapes and eyeball blood vessel patterns, fingerprints and finger veins etc.

Further, the query body inside information may be used for the identification query data, and the query surface information may be used for the verification query data. For example, palm vein information may be used for identification, and palm print information may be used for verification.

According to the biometric authentication system of the embodiment described above, accuracy and speed of one to many Authentication can be significantly improved, which is contrary to the general idea in the industry related to biometric authentication that the accuracy and speed of one to many Authentication are inferior to those of 1:1 Verification. The effects and features of the biometric authentication system of this embodiment are described below.

(1) Since a plurality of sets of independent biometric information having high authentication performance applicable to 1: 1 authentication are used for each of search (identification) and verification, the authentication performance is realized as a product of each of them. As a result, extremely high authentication performance can be realized.

(2) Since biometric information for searching (identification) and for verification are acquired from one image, convenience for the user is not impaired. Moreover, since a plurality of images is not required, it is possible to reduce system requirements.

(3) Since the palm vein information and the palm print information have same level high verification performance, even if the identification information and the verification information are used in reverse, similar high authentication performance can be realized.

(4) Since the verification template data to be verified can be narrowed down based on the identification biometric information, the speed of authentication is improved.

(5) Since the verification performance of the current palm vein is 0.0003% at the false acceptance rate, and the verification performance of the palm print is also 0.0003% at the false acceptance rate. Thus, the identification performance combining these is 1 in 100 billion at the false acceptance rate. As a result, it is possible to achieve the performance for a payment system by biometric authentication using one to many Authentication, which is an important application not been put into practical use yet.

1 : authentication image acquisition device
11 : authentication light source
12 : authentication image acquisition unit
13 : authentication image processing unit
131 : identification query image processing unit
1311 : palm print feature extraction unit
1312 : feature data conversion unit
132 : verification query image processing unit
1321 : vein feature extraction unit
1322 : feature data conversion unit
2 : template image acquisition device
21 : template light source
22 : template image acquisition unit
23 : template image processing unit
231 : identification template image processing unit
2311 : palm print feature extraction unit
2312 : feature data conversion unit
232 : verification template image processing unit
2321 : vein feature extraction unit
2322 : feature data conversion unit
24 : verification template data storage unit
3 : verification unit
4 : identification unit
41 : identification table generation unit
42 : identification table reference unit
43 : identification table storage unit
6 : mobile device
61 : display
62 : camera
8 : a hand of a user

## Claims

1. An individual authentication device comprising:

a query data acquisition unit configured to extract biometric information of human body surface as query surface information and biometric information of inside human body as query inside body information from one color query image obtained by photographing a human body of an individual, and
to generate identification query data from one of the query surface information and the query inside body information, and verification query data from the other of the query surface information and the query inside body information;

16

an identification unit configured to perform 1:N Identification with the identification query data and a plurality of already-registered identification registration data, and

to identify one or more verification registration data to be used in verification, the one or more verification registration data being associated with the identification registration data; and

a verification unit configured to authenticate a unique individual by performing 1:1 Verification with the identified one or more verification registration data using the degree of similarity with the verification query data; wherein the identification query data and identification registration data belong to a metric space,

the identification unit configure to identify one or more verification registration data to be used in the verification by performing the 1:N Identification based on the positional relationship in the metric space,

each of the identification registration data has an index value indicating that similar data in the metric space have a same index, and

the identification unit configure to convert the identification query data so that similar data in the metric space has a same index, and

to perform the 1 : N identification with identification registration data and the index generated by the conversion.

2. The device of claim 1, wherein
the identification query data and the verification query data have same information structure.

3. The device of claim 1 or clam 2, wherein
the 1:1 Verification is performed on all of the one or more verification registration data .

4. The device of any one of claim 1 to claim 3, wherein
the query surface information and the query inside body information are represented by line components,
the identification query data is data generated by Radon transform performed on one of the query surface information and the query inside body information represented by the line components, and
the verification query data is data generated by Radon transform performed on the other of the query surface information and the query inside body information represented by the line components.

5. The device of any one of claim 1 to claim 4, further comprising:

a registration unit configured to register the identification registration data associated with the verification registration data,
to extract biometric information of human body surface as template surface information and biometric information of inside human body as template inside body information from one color template image obtained by photographing a human body of an individual, and
to generate identification registration data corresponding to the individual from one of the template surface information and the template inside body information, and verification registration data from the other of the template surface information and the template inside body information; wherein
both the identification registration data and verification registration data have same information structure.

6. The device of claim5, wherein
the template surface information and the template inside body information are represented by line components,
the identification registration data is data generated by Radon transform performed on one of the template surface information and the template inside body information represented by the line components, and
the verification registration data is data generated by Radon transform performed on the other of the template surface information and the template inside body information represented by the line components.

7. The device of any one of claim 1 to claim 6, wherein
the positional relationship between the identification query data and the identification registration data is a positional relationship between these data and predetermined reference data.

8. The device of claim 7, wherein
the reference data is plural, and
the identification query data and identification registration data belong to each of groups based on the positional relationship between each reference data and the identification query data or identification registration data, and same group data have same index.

9. The device of any one of claim 1 to claim 8, wherein

the query image is an image of a palm of an authentication target individual, the query surface information is a palm print pattern extracted from the query image, and the query inside body information is a vein pattern extracted from the query image.

10. The device of claim 5 or claim 6, wherein
the template image is an image of a palm of a registration target individual, the template surface information is a palm print pattern extracted from the template image, and the template inside body information is a vein pattern extracted from the template image.

11. An individual authentication method comprising:

in a query data acquisition step, extracting biometric information of human body surface as query surface information and biometric information of inside human body as query inside body information from one color query image obtained by photographing a human body of an individual, and
generating identification query data from one of the query surface information and the query inside body information, and verification query data from the other of the query surface information and the query inside body information;
in an identification step, performing 1:N Identification with the identification query data and a plurality of already-registered identification registration data, and identifying one or more verification registration data to be used in verification, the one or more verification registration data being associated with the identification registration data; and
in a verification step, authenticating a unique individual by performing 1:1 Verification with the identified one or more verification registration data using the degree of similarity with the verification query data; wherein
the identification query data and identification registration data belong to a metric space,
in the identification step, identifying one or more verification registration data to be used in the verification by performing the 1:N Identification based on the positional relationship between the identification query data and identification registration data in the metric space,
each of the identification registration data has an index value indicating that similar data in the metric space have a same index, and
in the identification step, converting the identification query data so that similar data in the metric space has a same index, and
performing the 1 : N identification with identification registration data and the index generated by the conversion.

12. A computer program for causing a computer to perform each step in claim 11.

EP 3 734 548 A1

Fig. 1

2
Template Image Acquisition Device

1
Authentication Image Acquisition Device

4
Identification Unit

3
Verification Unit

19

Fig. 2

11
Authentication Light Source

12
Authentication image
Acquisition Unit

13
Authentication image
Processing Unit

Fig. 3

131

| Identification Query Image Processing Unit |

132

| Verification Query Image Processing Unit |

Fig. 4

Fig. 5

1311

Palm Print Feature
Extraction Unit

1312

Feature Date
Conversion Unit

Fig. 6

1321

Vein Feature
Extraction Unit

1322

Feature Date
Conversion Unit

Fig. 7

Fig. 8

Fig. 9

Palm Print Feature
Extraction Unit ........ 2311

Feature Data
Conversion Unit ........ 2312

Fig. 10

Vein Feature
Extraction Unit ........ 2321

Feature Data
Conversion Unit ........ 2322

Fig. 11

Identification Table
Generation Unit ........ 41

Identification Table
Reference Unit ........ 42

Identification Table
Storage Unit ........ 43

Fig. 12

| Process on template image | SA-1 |

↓

| Generate identification table | SA-2 |

↓

| Store verification template | SA-3 |

↓

| Acquire an authentication image | SA-4 |

↓

| Identification process | SA-5 |

↓

| Acquire verification template data | SA-6 |

↓

| Verification | SA-7 |

Fig. 13

Acquire a template image — SB-1

Generate identification template data Corresponding to vein shape — SB-3

Binarization — SB-5

Extract feature — SB-7

Coordinate transform — SB-9

Phase image transform — SB-11

Store identification template — SB-13

Generate identification template data Corresponding to palm print shape — SB-2

Binarization — SB-4

Extract feature — SB-6

Coordinate transform — SB-8

Phase image transform — SB-10

Generate identification table — SB-12

Fig. 14A

Identification template date
of extracted palm print pattern

Fig. 14B

Fig. 14C

Verification template date
of extracted vein pattern

Fig. 14D

Fig. 15

Fig. 16

```
┌──────────────────────────────────────────┐
│  Acquire ID and identification template   │────── SC-1
└──────────────────────────────────────────┘
                     │
                     ▼
┌──────────────────────────────────────────┐
│  Calculate identification table index value │──── SC-2
└──────────────────────────────────────────┘
                     │
                     ▼
┌──────────────────────────────────────────┐
│  Add ID to identification table index value │──── SC-3
└──────────────────────────────────────────┘
```

Fig. 17

EP 3 734 548 A1

Fig. 17

Fig. 18

EP 3 734 548 A1

Fig. 18

## INTERNATIONAL SEARCH REPORT

| International application No. |
| --- |
| PCT/JP2018/048095 |

A.  CLASSIFICATION OF SUBJECT MATTER
Int.Cl. G06T7/00(2017.01)i

According to International Patent Classification (IPC) or to both national classification and IPC

B.  FIELDS SEARCHED

Minimum documentation searched (classification system followed by classification symbols)
Int.Cl. G06T7/00, G06K9/00-9/82

| Documentation searched other than minimum documentation to the extent that such documents are included in the fields searched | |
| --- | --- |
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| A | JP 2010-218562 A (NEC CORP.) 30 September 2010, paragraphs [0062]-[0074] (Family: none) | 1-12 |
| A | WO 2010/044250 A1 (NEC CORP.) 22 April 2010, paragraphs [0032]-[0049] & US 2011/0200237 A1, paragraphs [0047]-[0064] | 1-12 |
| A | JP 2016-096987 A (HITACHI, LTD.) 30 May 2016, paragraphs [0047]-[0049] (Family: none) | 1-12 |
| A | WO 2012/014300 A1 (UNIVERSAL ROBOT INC.) 02 February 2012, paragraphs [0027]-[0038] (Family: none) | 1-12 |

☐  Further documents are listed in the continuation of Box C.        ☐  See patent family annex.

| | |
| --- | --- |
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| 12 March 2019 (12.03.2019) | 19 March 2019 (19.03.2019) |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

**EP 3 734 548 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010152706 A
- WO 20131365353 A
- JP 2010191573 A
- WO 2012014300 A
- WO 2013136533 A